Europäisches Patentamt

(19) European Patent Office

Office européen des brevets

(11) Publication number: **0 252 413 B1**

## (12) EUROPEAN PATENT SPECIFICATION

(45) Date of publication of patent specification:
13.11.91 Bulletin 91/46

(51) Int. Cl.⁵: **A61F 13/15**

(21) Application number: **87109381.1**

(22) Date of filing: **30.06.87**

(54) **Disposable diaper.**

(30) Priority: **11.07.86 JP 106530/86**

(43) Date of publication of application:
**13.01.88 Bulletin 88/02**

(45) Publication of the grant of the patent:
**13.11.91 Bulletin 91/46**

(84) Designated Contracting States:
**DE ES FR GB IT SE**

(56) References cited:
**DE-A- 3 319 043**
**US-A- 4 210 143**
**US-A- 4 381 781**

(73) Proprietor: **Kao Corporation**
**14-10, Nihonbashi Kayabacho 1-chome**
**Chuo-Ku Tokyo 103 (JP)**

(72) Inventor: **Toyoda, Harumitsu**
**117, Koedomachi**
**Utsunomiya-shi Tochigi (JP)**
Inventor: **Ito, Osamu**
**5-2, Motoimaizumimachi 6-chome**
**Utsunomiya-shi Tochigi (JP)**

(74) Representative: **Dickel, Klaus, Dipl.-Ing. et al**
**Julius-Kreis-Strasse 33**
**W-8000 München 60 (DE)**

# Description

The present device relates to a disposable diaper, and more specifically to a disposable diaper having such a structure as to improve the prevention of leakage in the waist portion and fit of the waist portion.

In general, for the purpose of preventing leakage, the abovementioned type of a conventional diaper has gathers provided in the crotch portion thereof, enabling the crotch portion of a wearer to be in close contact with a diaper. The structure of the diaper is such that an absorbent material is provided between a liquid-permeable top sheet and a liquid-impermeable back sheet and a fastener made of a pressure-sensitive adhesive tape is provided on the main body constituted of the above-mentioned main constituents for fixing the diaper to the body of a baby.

Such a diaper well fits around the thighs of a wearer and closely contacts with the crotch of the wearer. Since the waist portion is merely fixed to the back sheet of the main body of the diaper with a rigid pressure-sensitive adhesive tape, however, excreta will sometimes leak from the waist portion when the baby lies. To obviate this defect, there is proposed in, for example, Japanese Patent Laid-Open No. 87,303/1983 a diaper comprising a stretchable member attached onto the upper portion of an absorbent material on the side of a pressure-sensitive adhesive tape along the crosswise direction of a back sheet. In this type of diaper, however, the pressure-sensitive adhesive tape is also constituted of a stretchable material, thus narrowing the tolerance limits of adhesion required of the tape during the use of the diaper. Thus, a grave difficulty is encountered in preparing a tape. Further, since the stretchable member is outside the back sheet, the waist portion is merely pressed. Thus, the effect of preventing leakage of urine from the waist portion is small.

There are also proposed diapers in Japanese Utility Model Publication No. 7,709/1985 and Japanese Patent Laid-Open No. 173,101/1985. In a diaper disclosed in Japanese Utility Model Publication No. 7,709/1985, films are provided in the waist portion, namely in both the end portions in the lengthwise direction in such a way that the absorbent material is partially covered with the films. Although leakage of urine through the absorbent material is somewhat prevented in such a diaper, leakage of excreta positioned on the top sheet cannot be prevented and the fit cannot be satisfactory. On the other hand, in a diaper disclosed in Japanese Patent Laid-Open No. 173,101/1985, a stretchable member is positioned only inside the back sheet. Thus, the effect of preventing leakage through the waist portion is small as in the case of the diaper disclosed in Japanese Patent Laid-Open No. 87,303/1983.

The devisers of the present device have made intensive investigations with a view to obviate the defects of the conventional disposable diaper. As a result, they have found that provision of a soft and elastic liquid-impermeable barrier sheet between a top sheet and back sheet on the side in the lengthwise direction on which side a fastener such as a pressure-sensitive adhesive tape is provided and fixation of at least one end portion of the barrier sheet to a back sheet with the other end portion positioned between an absorbent material and a top sheet can provide softness of the waist portion, close contact with a relatively broad contact area, easiness in attachment and detachment of the pressure-sensitive adhesive tape due to stretching and shrinkage thereof together with the back sheet, thus effectively preventing the leakage from the waist portion.

Brief Description of the Drawings:

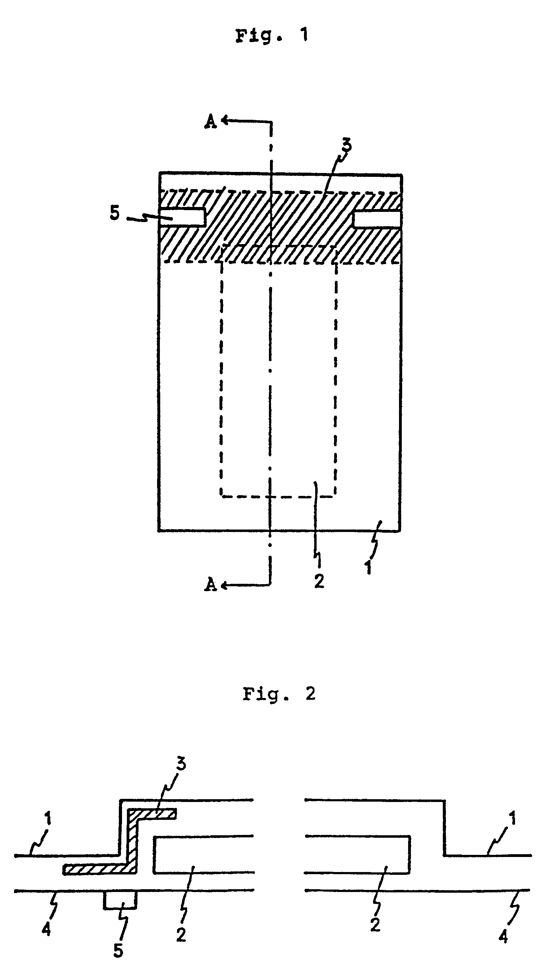

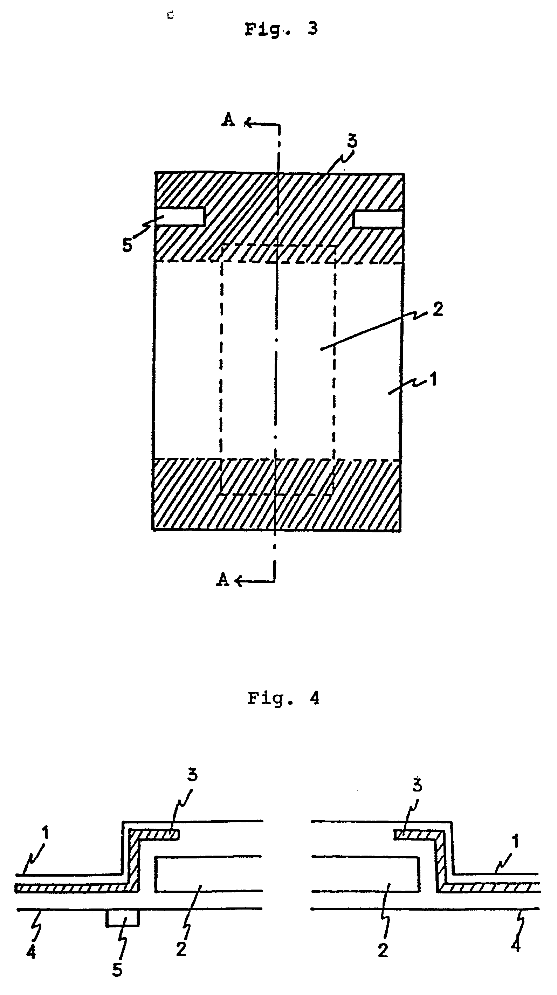

Fig. 1 is a plan view of an example of the disposable diaper of the present device; Fig. 2 is a crosssectional view thereof along the line A-A; Fig. 3 is a plan view of another example of the disposable diaper of the present device; and Fig. 4 is a crosssectional view thereof along the line A-A.

1 :  top sheet
2 :  absorbent material
3 :  barrier sheet
4 :  back sheet
5 :  fastener (pressure-sensitive adhesive tape)

A disposable diaper of the invention has a rectangular plane surface and comprises a liquid-permeable top sheet, a liquid-impermeable back sheet, an absorbent material provided between the two sheets, a pair of fasteners provided around one end of the diaper in relation to the longitudinal direction to fasten the one end to the other and an elastic liquid-impermeable barrier sheet provided between the top sheet and the back sheet at least on the end of the diaper having said fasteners, said barrier sheet being fixed at one end at least onto the back sheet and at the other end located between the absorbent material and the top sheet.

It is preferable that the said back sheet and the barrier sheet each have an elongation of 20 percent or larger at a load of 200 g/cm at the lateral direction of the diaper. It is advantageous from the practical point of view that the back sheet and the barrier sheet have almost the same elongation as each other.

Specifically, in accordance with the present device, there is provided a disposable diaper which is substantially rectangular and comprises a liquid-permeable top sheet, a liquid-impermeable back sheet, an absorbent material positioned between the two sheets, and a pair of fasteners provided in one end portion in the lengthwise direction, characterized in that an elastic liquid-impermeable barrier sheet is provided between said top sheet and said back sheet in the end portion on the side in the lengthwise direction on which side said pair of fasteners are provided, and that at least one end of said barrier sheet is fixed to said back sheet while the other end of said barrier sheet is positioned between said absorbent material and said top sheet.

In the disposable diaper of the present device, since the barrier sheet is provided in the waist portion, the fit of the waist portion is good, thus preventing leakage of excreta from waist portion, while leakage from the waist portion due to oozing of urine absorbed in the absorbent material can also be prevented since one end portion of the barrier sheet is provided in such a way to cover part of the absorbent material.

In order that the barrier sheet involved in the present device effectively acts on the waist portion, the width of a portion where it does not overlap the absorbent material is preferably 20 mm or more. In the disposable diaper of the present device, the elongation of each of the back sheet and the barrier sheet in the crosswise direction is 20% or more under a load of 200 g/cm because the above-mentioned barrier sheet and the back sheet fixing the barrier sheet thereto stretch and shrink together. It is more preferred that the barrier sheet have an air permeability.

In the present device, the "substantially rectangular" disposable diaper include one having a longitudinally extend form like a sandglass.

The fastener (pressure-sensitive adhesive tape) of the disposable diaper of the present device is preferably bonded to the barrier sheet through the top sheet or the back sheet. If so, the force applied to the fastener is directly transmitted to the barrier sheet, which therefore effectively acts on the waist.

(Example)

Examples of the present device will now be described in detail with reference to the drawings. Fig. 1 is a plan view of an example of the disposable diaper of the present device, and Fig. 2 is a crosssectional view thereof along the line A-A. Fig. 3 is a plan view of another example of the disposable diaper of the present device, and Fig. 4 is a crosssectional view thereof along the line A-A.

In a disposable diaper as shown in Figs. 1 and 2, a barrier sheet 3 is positioned between a top sheet 1 and a back sheet 4 in the end portion in the lengthwise direction of an absorbent material on the side on which a fastener 5 is provided. One end portion of the barrier sheet is positioned along the crosswise direction of the back sheet and between the top sheet 1 and absorbent material 2, namely on the absorbent material 2. The barrier sheet is fixed in such a way that the portion of the barrier sheet not overlapping the absorbent material is 20 mm or more, or that the overlapping portion on the absorbent material 2 does not prevent the stretching of the barrier sheet 3 at least in the crosswise direction as much as possible. Fixation may be made with an adhesive such as a rubber or ethylenevinyl acetate copolymer (EVA) hot-melt adhesive.

The diaper of the present device may have barrier sheets 3 positioned between a top sheet 1 and a back sheet 4 and in both end portions in the lengthwise direction of an absorbent material as in an example shown in Figs. 3 and 4.

Since the disposable diaper of the present device is constituted as described above, the barrier sheet 3 stretches and shrinks together with the back sheet 4 depending on the size of the waist portion of a wearer. Therefore, the fastener is easily attached and detached in the use of the diaper. The disposable diaper absorbs excreta, thereby preventing leakage from the waist portion when the baby lies. Further, the fit of the waist portion is improved.

Any sheet which shows a rubber elasticity can be used as the barrier sheet capable of exhibiting such effects. Examples of the material of such a sheet include styrene-butylene-styrene rubber (SBS), styreneisoprene-styrene rubber (SIS), styrene-ethylene-butadiene-styrene rubber (SEBS), SEBS/SIS, urethane, ethylene-vinyl acetate copolymer (EVA) and composites thereof.

An air-permeable sheet is more preferred. A porous film and α non-woven fabric are preferred for securing air permeability. A moisture-permeable urethane film or the like may be used.

The important points are the width of a portion of the barrier sheet not overlapping the absorbent material and an elongation in the crosswise direction of the back sheet constituting the diaper together with the absorbent material.

From the viewpoint of pressure against a wearer, it is important that the barrier sheet be wide and have a low elasticity. Therefore, the barrier sheet involved in the present device is desired to be combined with a back sheet having an elongation of 20% or more under a load of 200 g/cm for securing effective stretching and shrinkage in the waist portion even with the use of a conventional rigid pressure-sensitive adhesive tape. When the elongation is less than 20%, the non-woven fabric may be broken without manifesting the above-mentioned elastic effect, the back sheet may also be broken, and the stretching and shrinking effect of the waist portion may not be manifested so that the main body of the diaper may be snatched away from the pressure-sensitive adhesive tope portion, thus spoiling the fastening function of the pressure-sensitive adhesive tape as the essential function of the diaper.

The use of the disposable diaper of the present device provides effects which cannot be desired by conventional diapers and hence is very beneficial.

## Claims

1. A disposable diaper which has a rectangular plane surface and comprises a liquid-permeable top sheet (1), a liquid-impermeable back sheet (4), an absorbent material (2) provided between the two sheets, a pair of fasteners (5) provided around one end of the diaper in relation to the longitudinal direction to fasten the one end to the other end and an elastic liquid-impermeable barrier sheet (3) provided between the top sheet (1) and the back sheet (4) at least on the end of the diaper having said fasteners (5), said barrier sheet (3) being fixed at one end at least to the back sheet (4) and at the other end located between the absorbent material (2) and the top sheet (1), characterized by said back sheet (4) and said barrier sheet (3) have an elongation each of 20 % or larger at a load of 200 g/cm in the lateral direction of the diaper.

2. A disposable diaper as claimed in claim 1, in which the back sheet (4) and the barrier sheet (3) have almost the same elongation.

## Patentansprüche

1. Wegwerfwindel mit einer rechteckförmigen, ebenen Oberfläche sowie einer flüssigkeitsdurchlässigen Oberschicht (1), einer flüssigkeitsundurchlässigen Rückschicht (4), einem absorbierenden Material (2), das zwischen den beiden Schichten vorgesehen ist, einem Paar von Befestigungselementen (5), die um ein Ende der Windel herum in bezug auf die Längsrichtung angeordnet sind, zur Befestigung des einen Endes an dem anderen Ende, und einer elastischen, flüssigkeitsundurchlässigen Sperrschicht (3) zwischen der Oberschicht (1) und der Rückschicht (4), wobei mindestens an dem Ende der Windel, das die Befestigungselemente (5) trägt, die Sperrschicht (3) an einem Ende zumindest der Rückschicht (4) befestigt ist, während sich das andere Ende zwischen dem absorbierenden Material (2) und der Oberschicht (1) befindet, dadurch gekennzeichnet, daß die Rückschicht (4) und die Sperrschicht (3) eine Dehnung von jeweils 20 % oder mehr bei einer Belastung von 200 g/cm in seitlicher Richtung der Windel besitzen.

2. Wegwerfwindel nach Anspruch 1, dadurch gekennzeichnet, daß die Rückschicht (4) und die Sperrschicht (3) nahezu die gleiche Dehnung besitzen.

## Revendications

1. Couche jetable ayant une surface rectangulaire plane et comprenant une feuille supérieure (1) perméable aux liquides, une feuille inférieure (4) imperméable aux liquides, une matière absorbante (2) placée entre les deux feuilles, une paire d'attaches (5) placées autour d'une prer re extrémité de la couche par rapport au sens lon itudinal pour attacher la première extrémité à l'autre extrémité et une feuille formant barrage (3), élastique, imperméable aux liquides, placée entre la feuille supérieure (1) et la feuille inférieure (4) au moins sur l'extrémité de la couche portant lesdites attaches (5), ladite feuille formant barrage (3) étant fixée, au niveau d'une extrémité au moins, à la feuille inférieure (4) et placée, à l'autre extrémité, entre la matière absorbante (2) et la feuille supérieure (1), caractérisée en ce que ladite feuille inférieure (4) et ladite feuille formant barrage (3) ont chacune un allongement de 20 % ou plus à une charge de 200g/cm dans le sens latéral de la couche.

2. Couche jetable selon la revendication 1, dans laquelle la feuille inférieure (4) et la feuille formant barrage (3) ont presque le même allongement.

Fig. 1

Fig. 2

Fig. 3

Fig. 4